(19) Europäisches Patentamt
European Patent Office
Office européen des brevets

(11) **EP 4 428 868 A1**

(12) **EUROPEAN PATENT APPLICATION**

(43) Date of publication:
**11.09.2024 Bulletin 2024/37**

(21) Application number: **24160976.7**

(22) Date of filing: **01.03.2024**

(51) International Patent Classification (IPC):
**G16H 10/60** (2018.01)   **G16H 20/30** (2018.01)
**G16H 20/00** (2018.01)

(52) Cooperative Patent Classification (CPC):
**G16H 10/60; G16H 20/00; G16H 20/30;**
G16H 50/30; G16H 80/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.03.2023 US 202318181733**

(71) Applicant: **Icon Clinical Research Limited
Dublin 18 (IE)**

(72) Inventors:
• **FOX, Ronan
  Dublin (IE)**
• **KELLY, Sean
  Dublin (IE)**
• **O'LEARY, Thomas
  Dublin (IE)**

(74) Representative: **Weldon O'Brien Ltd.
Shannon Lodge
Casement Road
Bandon
County Cork, P72 TN24 (IE)**

(54) **SYSTEMS AND METHODS FOR PROVENANCE AND DATA INTEGRITY MONITORING**

(57)     Systems and methods for determining data integrity are disclosed. The method may include determining that a first reading of a user characteristic is requested; causing a user device associated with the user to output one or more instructions for collecting the first reading; causing one or more first sensor associated with the user device to collect the first reading; causing one or more second sensor associated with the user device to collect a second reading; determining data indicative of user adherence to the instructions; determining a protocol deviation value; determining whether there is a protocol deviation for the first reading; and saving the protocol deviation determination to a storage device.

250

**FIG. 2B**

EP 4 428 868 A1

**Description**

TECHNICAL FIELD

Various embodiments of the present disclosure relate generally to remotely managing and determining data integrity for a clinical trial. More specifically, particular techniques of the present disclosure relate to systems and methods for determining a protocol deviation of a first reading by a first sensor.

BACKGROUND

Generally, clinical trials are becoming increasingly decentralized and may involve participants collecting samples, recordings, etc. without the supervision of a medical professional. Participants may be provided with written instructions, e.g., on an instruction card, but may fail to properly follow the instructions, which may result in improperly collected samples, recordings, etc. While some errors in collection may not alter the data, other errors may affect the data to a statistically significant degree. Further, under conventional techniques, researchers may be unable to determine the extent to which the provided instructions were properly followed, which may limit the extent to which the researcher may be able to distinguish an insignificant error from a significant error.

Conventional techniques, including the foregoing, generally increase the risk of inaccurate and/or unreliable data, which may affect the veracity of the study conclusions. Such factors may be exacerbated by the study participants no longer being local to the study and thus possibly outside the range of personal supervision. Without supervision by a medical professional, study participants may struggle to collect the requisite measurements, forget to take the measures, improperly take the measures, take too few or too many measures, and/or may take measures at improper times or locations or in the presence of factors that may skew results. These factors may weigh on the accuracy and/or reliability of the study data, which, if not accounted for, may lead to inaccurate, unreliable, and/or improper study conclusions.

The present disclosure is directed to addressing one or more of these above-referenced challenges. The background description provided herein is for the purpose of generally presenting the context of the disclosure. Unless otherwise indicated herein, the materials described in this section are not prior art to the claims in this application and are not admitted to be prior art, or suggestions of the prior art, by inclusion in this section.

SUMMARY

The invention provides in various examples methods and systems as set out in the appended claims 1 to 20. According to certain aspects of the disclosure, methods and systems are disclosed for monitoring data provenance and determining data integrity. Each of the aspects of the disclosure herein may include one or more of the features described in connection with any of the other disclosed aspects.

According to one example of the present disclosure, a method for determining data integrity may be described. The method may include determining, via a processor, that a first reading of a user characteristic is requested; causing a user device associated with the user to output one or more instructions for collecting the first reading, the instructions based on a predetermined protocol; causing one or more first sensor associated with the user device to collect the first reading; causing one or more second sensor associated with the user device to collect a second reading; determining, via the processor, based on one or more of the first reading, the second reading, or metadata associated with one or more of the first sensor or the second sensor, data indicative of user adherence to the instructions; determining, via the processor, a protocol deviation value for the first reading based on the second reading and the data indicative of user adherence to the instructions; determining, via the processor, whether there is a protocol deviation for the first reading based on a comparison of the determined protocol deviation value with a deviation threshold value; and saving the protocol deviation determination to a storage device.

According to another example of the present disclosure, a system for determining data integrity may be described. The system may include at least one memory storing instructions; and at least one processor configured to execute the instructions to perform operations for determining data integrity, the operations including: determine that a first reading of a user characteristic is requested; cause a user device associated with the user to output one or more instructions for collecting the first reading, the instructions based on a predetermined protocol; cause one or more first sensor associated with the user device to collect the first reading; cause one or more second sensor associated with the user device to collect a second reading; determine based on one or more of the first reading, the second reading, or metadata associated with one or more of the first sensor or the second sensor, data indicative of user adherence to the instructions; determine a protocol deviation value for the first reading based on the second reading and the data indicative of user adherence to the instructions; determine whether there is a protocol deviation for the first reading based on a comparison of the determined protocol deviation value with a deviation threshold value; and save the determined protocol deviation to a storage device.

According to another example of the present disclosure, a computer-implemented method for determining data integrity may be described. The computer-implemented method may include determining, via a processor, that a first reading of a user characteristic is requested; determining, via the processor, a user biometric identification based on a comparison of a biometric measure with a biometric threshold, wherein the biometric measure is determined based on a comparison of a biometric reading with a patient-specific biometric profile; causing a user device associated with the user to output one or more instructions for collecting the first reading, the instructions based on a predetermined protocol; causing one or more first sensor associated with the user device to collect the first reading; causing one or more second sensor associated with the user device to collect a second reading; determining, via the processor, based on one or more of the first reading, the second reading, or metadata associated with one or more of the first sensor or the second sensor, data indicative of user adherence to the instructions; determining, via the processor, a protocol deviation value for the first reading based on the second reading and the data indicative of user adherence to the instructions; determining, via the processor, whether there is a protocol deviation for the first reading based on a comparison of the determined protocol deviation value with a deviation threshold value; determining, via the processor, a measurement validity of the first reading based whether a validity measure exceeds a validity measure threshold, wherein the validity measure is determined based on a comparison of the first reading with a validity range specific to the user; obtaining a difficulty score for the first reading based on historical information associated with the first reading, the historical information including one or more of historical information regarding the data indicative of user adherence to the instructions or historical information regarding a number of attempts needed to record the first reading; appending a risk indicator for each of the user biometric identification, the protocol deviation value, the measurement validity, and the difficulty score to the first reading; and determining an overall risk associated with the first reading, the overall risk determined based on the risk indicators for each of the user biometric identification, the protocol deviation value, the measurement validity, and the difficulty score.

According to one example of the present disclosure, a computer-implemented method performed by a processor for determining data provenance may include: determining, via a processor, that a first reading of a user characteristic is requested; causing a user device associated with the user to output one or more instructions for collecting the first reading, the one or more instructions being based on a predetermined protocol; causing a first sensor associated with the user device to collect the first reading; determining a user biometric identification in response to determining that the first reading of a user is requested; generating a biometric reading based on one or more of the first reading or metadata associated with the first sensor; and determining a biometric measure based on a comparison of the biometric reading with a patient-specific biometric profile.

According to one example of the present disclosure, a computer-implemented method performed by a processor for determining data validity may include: determining, via a processor, that a first reading of a user characteristic is requested; causing a user device associated with the user to output one or more instructions for collecting the first reading, the instructions being based on a predetermined protocol; causing a first sensor associated with the user device to collect the first reading; causing a second sensor associated with the user device to collect a second reading; and determining a measurement validity of the first reading based on the second reading.

According to one example of the present disclosure, a computer-implemented method performed by a processor for setting up a sensor package on a user device may include: determining, via a processor, that a user has been enrolled in a clinical trial; determining, based on metadata associated with the clinical trial, one or more sensor readings requiring collection for the clinical trial; determining based on the one or more sensor readings, one or more sensors usable to collect the one or more sensor readings; obtaining one or more software setup module for the one or more sensors; generating the sensor package based on the one or more software setup modules; and deploying the sensor package to the user device, the deploying configured to cause the user device to configure the user device to operate the one or more sensors.

According to one example of the present disclosure, a computer-implemented method performed by a processor for automatic generation of user instructions related to a software setup package required for a clinical study may include: determining, via a processor, that a user has been enrolled in a clinical trial; deploying the software setup package on a user device; obtaining data from the software setup package that identifies one or more sensor package associated with the clinical trial, the sensor package including one or more sensors; and providing, through a user interface of the user device, based on the software setup package, instructions associated with the one or more sensors from the one or more sensor package.

According to one example of the present disclosure, a computer-implemented method performed by a processor for determining correlations between one or more sensors in a clinical study may include: receiving as input to a processor, a plurality of sensor readings from a plurality of different sensors; determining that a subset of the sensor readings are correlated; based on the determined correlation, determining a relationship between a subset of the sensors.

It is to be understood that both the foregoing general description and the following detailed description are exemplary and explanatory only and are not restrictive of the disclosed embodiments, as claimed.

The present disclosure furthermore relates to the following aspects.

We describe a computer-implemented method performed by a processor for determining data provenance, the method comprising: determining, via a processor, that a first reading of a user characteristic is requested; causing a user device associated with the user to output one or more instructions for collecting the first reading, the one or more instructions being based on a predetermined protocol; causing a first sensor associated with the user device to collect the first reading; determining a user biometric identification in response to determining that the first reading of a user is requested; generating a biometric reading based on one or more of the first reading or metadata associated with the first sensor; and determining a biometric measure based on a comparison of the biometric reading with a patient-specific biometric profile.

In some examples, determining the user biometric identification includes comparing the biometric measure with a biometric threshold.

In some examples, upon determining the biometric measure is not within a biometric threshold, instructing the user to repeat a collection process of the first reading.

In some examples, the method further includes: causing a second sensor associated with the user device to collect a second reading; generating a second biometric reading based on the second reading or metadata associated with the second sensor; and determining a biometric measure based on a comparison of the second reading with the patient-specific biometric profile.

In some examples, the comparison of the biometric reading with a patient-specific biometric profile includes: obtaining a baseline biometric fingerprint of the user; obtaining, based on the biometric reading, a reading of a user's biometric fingerprint; and comparing the baseline biometric fingerprint of the user to the reading of the user's biometric fingerprint to verify that the user is a correct patient.

In some examples, the baseline biometric fingerprint is updated over a course of a clinical trial as a user's characteristic changes.

In some examples, a machine learning model is used to generate the patient-specific biometric profile.

We also describe a computer-implemented method performed by a processor for determining data validity, the method comprising: determining, via a processor, that a first reading of a user characteristic is requested; causing a user device associated with the user to output one or more instructions for collecting the first reading, the instructions being based on a predetermined protocol; causing a first sensor associated with the user device to collect the first reading; causing a second sensor associated with the user device to collect a second reading; and determining a measurement validity of the first reading based on the second reading.

In some examples, the method further includes: generating a validity range specific to the user based on one or more of the first reading, the second reading, or metadata associated with one or more of the first sensor or the second sensor; and determining a validity measure based on a comparison of the first reading with the validity range specific to the user.

In some examples, determining the measurement validity includes determining whether the validity measure exceeds a validity measure threshold.

In some examples, the method further includes: using a sensor measurement data package to determine the first reading the second reading, and metadata associated with the first reading and/or second reading.

In some examples, the first sensor is a fitness application sensor and the second sensor is an EMR sensor.

In some examples, the first reading is heart rate data and the second reading is historical patient heart rate rates.

In some examples, the measurement validity includes determining whether the user adhered to the instructions by: evaluating the first reading, the second reading to determine whether a protocol deviation occurred based on a determination of one or more of whether a user selected correct buttons according to the one or more instructions, followed required or necessary steps according to the one or more instructions, or provided proof that steps according to the one or more instructions were followed.

In some examples, the first reading includes heart rate data, the second reading includes GPS data, wherein a protocol for a clinical study requires that the first reading be taken while the user is stationary, wherein the GPS data confirms whether the protocol deviation occurred.

We also describe a computer-implemented method performed by a processor for setting up a sensor package on a user device, the method comprising: determining, via a processor, that a user has been enrolled in a clinical trial; determining, based on metadata associated with the clinical trial, one or more sensor readings requiring collection for the clinical trial; determining based on the one or more sensor readings, one or more sensors usable to collect the one or more sensor readings; obtaining one or more software setup module for the one or more sensors; generating the sensor package based on the one or more software setup modules; and deploying the sensor package to the user device, the deploying configured to cause the user device to configure the user device to operate the one or more sensors.

In some examples, deploying the sensor package further includes: updating any sensor software required for operating the one or more sensors.

In some examples, determining the one or more sensors includes identifying at least one sensor having a predetermined relationship with at least one other sensor and/or at least one of the one or more readings for the clinical trial.

In some examples, the deploying includes: automatically calibrating at least one of the one or more sensors.

In some examples, the method further includes: obtaining instructions associated with setup and/or use of the one or more sensors, and causing the user device to output the obtained instructions to the user.

In some examples, the deploying includes establishing a data connection between the user device and at least one system remote from the user device.

In some examples, the user device is a cellphone.

We also describe a computer-implemented method performed by a processor for automatic generation of user instructions related to a software setup package required for a clinical study, the method comprising: determining, via a processor, that a user has been enrolled in a clinical trial; deploying the software setup package on a user device; obtaining data from the software setup package that identifies one or more sensor package associated with the clinical trial, the sensor package including one or more sensors; and providing, through a user interface of the user device, based on the software setup package, instructions associated with the one or more sensors from the one or more sensor package.

In some examples, the method further includes: identifying which of the one or more sensors of the sensor package do not require any user setup and/or maintenance.

In some examples, the instructions include instructions on connecting, calibrating, and/or validating the one or more sensors.

In some examples, the methodfurther includes: determining, based on the data from the software setup package, a list of required sensors; and outputting the list of required sensors to the user.

In some examples, the method further includes: determining, based on the data from the software setup package, instructions to a patient for how to obtain readings from the one or more sensors of the sensor package; and outputting the instructions to the user through the user interface.

In some examples, the software setup package includes data on how the one or more sensors relate to one another, and outputting the data on how the one or more sensors relate to one another to the user through the user interface.

In some examples, the method further includes: obtaining from the software setup package a step-by-step guidance on how to obtain a reading via the one or more sensors; and outputting the step-by-step guidance on how to obtain a reading to the user through the user interface.

We also describe a computer-implemented method performed by a processor for determining correlations between one or more sensors in a clinical study, the method comprising: receiving as input to a processor, a plurality of sensor readings from a plurality of different sensors; determining that a subset of the sensor readings are correlated; based on the determined correlation, determining a relationship between a subset of the sensors.

In some examples, the correlation is determined by using one or more algorithm configured to mathematically define influences between the subset of sensors.

In some examples, the relationship is usable to validate a reading from one or more sensors in the subset by evaluating reading from the other sensor(s) in the subset.

In some examples, the correlation is determined by using one or more machine learning techniques.

In some examples, the method further includes: using the relationship between the subset of sensors to automatically configure a second algorithm.

In some examples, a first sensor in the subset of sensors is a heart rate sensor and a second sensor in the subset of sensors is a pace data sensor.

In some examples, at least one sensor is included in multiple subsets of sensors, such that the at least one sensor has multiple different relationships with different sensors in the at least one sensor package.

In some examples, the determining of the relationship includes receiving a defined relationship between the subset of sensors.

We also describe a computer-implemented method for determining data integrity, the method comprising: determining, via a processor, that a first reading of a user characteristic is requested; causing a user device associated with the user to output one or more instructions for collecting the first reading, the instructions based on a predetermined protocol; causing one or more first sensor associated with the user device to collect the first reading; causing one or more second sensor associated with the user device to collect a second reading; determining, via the processor, based on one or more of the first reading, the second reading, or metadata associated with one or more of the first sensor or the second sensor, data indicative of user adherence to the instructions; determining, via the processor, a protocol deviation value for the first reading based on the second reading and the data indicative of user adherence to the instructions; determining, via the processor, whether there is a protocol deviation for the first reading based on a comparison of the determined protocol deviation value with a deviation threshold value; and saving the protocol deviation determination to a storage device.

In some examples, the predetermined protocol further includes a schedule for requesting the first reading; and the determining that a first reading of a user characteristic is requested is based on the predetermined protocol.

In some examples, the method further comprises: hashing the metadata to at least one of the first reading or the instructions.

In some examples, the processor is configured to, upon determining that there is no protocol deviation, append a risk indicator to the first reading, the risk indicator based on the determined protocol deviation value.

In some examples, the processor is configured to, upon determining that there is a protocol deviation, cause the one or more first sensor associated with the user device to collect a further first reading.

In some examples, the method further comprises: determining a measurement validity of the first reading via one or more of the first sensor or the second sensor.

In some examples, the method further comprises: generating a validity range specific to the user based on one or more of the first reading, the second reading, or metadata associated with one or more of the first sensor or the second sensor; and determining a validity measure based on a comparison of the first reading with the validity range specific to the user, wherein determining the measurement validity includes determining whether the validity measure exceeds a validity measure threshold.

In some examples, the method further comprises: determining a user biometric identification in response to determining that the first reading of a user is requested.

In some examples, the method further comprises: generating a biometric reading based on one or more of the first reading, the second reading, or metadata associated with one or more of the first sensor or the second sensor; and determining a biometric measure based on a comparison of the biometric reading with a patient-specific biometric profile, wherein determining the user biometric identification includes comparing the biometric measure with a biometric threshold.

In some examples, the method further comprises: obtaining a difficulty score for the first reading based on historical information associated with the first reading, the historical information including one or more of historical information regarding the data indicative of user adherence to the instructions or historical information regarding a number of attempts needed to record the first reading; and appending a further risk indicator to the first reading, the risk indicator based on the obtained difficulty score.

In some examples, the method further comprises: determining an overall risk associated with the first reading, the overall risk determined based on one or more factors with associated weightings.

We also describe a non-transitory CRM storing instructions executable by at least one processor to perform the method of any of the example

## BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.

FIG. 1A depicts an exemplary environment for collecting and/or influencing one or more readings, according to one or more embodiments.

FIG. 1B depicts another exemplary environment for collecting and/or influencing one or more readings, according to one or more embodiments.

FIG. 1C depicts an exemplary environment for contextualizing data, according to one or more embodiments.

FIG. 2A depicts an exemplary environment for monitoring and determining data provenance, according to one or more embodiments.

FIG. 2B depicts an exemplary sensor application framework, according to one or more embodiments.

FIG. 3 depicts an exemplary method for determining data integrity, according to one or more embodiments.

FIG. 4 depicts an exemplary schematic for sensor setup package deployment, according to one or more embodiments.

FIG. 5 depicts an exemplary method for sensor setup package deployment, according to one or more embodiments

FIG. 6A depicts an exemplary method for normalizing data, according to one or more embodiments.

FIG. 6B depicts an exemplary graph of normalizing data utilizing techniques discussed herein, according to one or more embodiments.

FIG. 7 depicts a simplified functional block diagram of a computer, according to one or more embodiments.

## DETAILED DESCRIPTION OF EMBODIMENTS

According to certain aspects of the disclosure, methods and systems are disclosed for monitoring data provenance, determining data integrity, and/or other tasks that may be related to remotely managing a clinical trial or similar operation. These methods and system may be usable to determine the accuracy and/or reliability of data collected during, for example, clinical trials. As will be discussed in more detail below, in various embodiments, systems and methods are described that incorporate features such as protocol deviation determination, measurement validity determination, patient identification, difficulty determination, overall risk determination, and/or data normalization.

Reference to any particular activity is provided in this disclosure only for convenience and not intended to limit the disclosure. The disclosure may be understood with reference to the following description and the appended drawings, wherein like elements are referred to with the same reference numerals.

The terminology used below may be interpreted in its broadest reasonable manner, even though it is being used in conjunction with a detailed description of certain specific examples of the present disclosure. Indeed, certain terms may even be emphasized below; however, any terminology intended to be interpreted in any restricted manner will be overtly and specifically defined as such in this Detailed Description section. Both the foregoing general description and the following detailed description are exemplary and explanatory only and are not restrictive of the features, as claimed.

In this disclosure, the term "based on" means "based at least in part on." The singular forms "a," "an," and "the" include plural referents unless the context dictates otherwise. The term "exemplary" is used in the sense of "example" rather than "ideal." The terms "comprises," "comprising," "includes," "including," or other variations thereof, are intended to cover a non-exclusive inclusion such that a process, method, or product that comprises a list of elements does not necessarily include only those elements, but may include other elements not expressly listed or inherent to such a process, method, article, or apparatus. The term "or" is used disjunctively, such that "at least one of A or B" includes, (A), (B), (A and A), (A and B), etc. Relative terms, such as, "substantially" and "generally," are used to indicate a possible variation of $\pm 10\%$ of a stated or understood value.

It will also be understood that, although the terms first, second, third, etc. are, in some instances, used herein to describe various elements, these elements should not be limited by these terms. These terms are only used to distinguish one element from another. For example, a first contact could be termed a second contact, and, similarly, a second contact could be termed a first contact, without departing from the scope of the various described embodiments. The first contact and the second contact are both contacts, but they are not the same contact.

As used herein, the term "if' is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Terms like "provider," "medical provider," or the like generally encompass an entity, person, or organization that may seek information, resolution of an issue, or engage in any other type of interaction with a patient, e.g., to provide medical care, medical intervention or advice, or the like. Terms like "patient," "client," "participant," "study participant" or the like generally encompass any person or entity who is obtaining information, seeking resolution of an issue, or engaging in any other type of interaction with a medical provider, e.g., to receive such care, information, or the like. Terms like "user" generally encompass any person or entity that may obtain information, resolution of an issue, purchase of a product, or engage in any other type of interaction with a provider or patient, whereby the user may be the medical provider or the patient as the case may be. For example, an individual that collects data on themselves may be both a patient and a user.

In some instances, a clinical trial may be conducted in a remote and/or decentralized manner. Such a clinical trial structure may be desirable for myriad reasons. For example, a remote study may be conducted across a large geographical area with a large sample size of patients, thereby improving the accuracy and reliability of any conclusions drawn in the study. In another example, decentralized studies may allow research to continue largely uninterrupted by external events, such as pandemics, states of emergency, etc. In a further example, decentralized studies may be more convenient for participants and researchers. In a remote study, participants may not need to travel to a medical provider as often for the collection of samples, readings, etc. Rather, the participant may be able to take the sample, reading, etc. when necessary and with minimal interruption to their day. As a further benefit, a greater number of data points may be collected, since participants may be less likely to skip or miss a session due to the inconvenience of travel.

However, such a clinical trial structure may require that participants collect their own samples, readings, etc., e.g., collect and record their own heart rate, which may prove challenging for the participants. Participants may struggle to collect the data, may not collect the data properly or at all, or may encounter other challenges when collecting their data. Improperly collected data may affect study conclusions based on the data. For example, improperly collected blood pressures may cause researchers to believe a blood pressure drug may be more or less effective than it may actually be. Thus, it may be beneficial to improve methods for determining data integrity, e.g., by measuring various risk dimensions associated with data collection to determine an overall data risk.

In an exemplary use case, a remote and/or decentralized clinical trial (hereinafter "clinical trial") may be developed, e.g., in order to evaluate an effect or effectiveness of a medical intervention such as a blood pressure drug. Developing the trial may include, for example, selecting patients for the trial, and determining what data needs to be gathered. For example, for the blood pressure drug evaluation, patients' blood pressure may be a desired measure. Additionally, it may be desirable to obtain additional measures, e.g., to assist in validating data provenance and integrity as discussed in further detail below. As part of the clinical trial, the patient may need to collect one or more readings of the desired data without medical supervision. Based on the requirements of the clinical trial, e.g., what readings the patient may need to collect, a Software Setup Package (SSP) may be deployed to a user device, e.g., a cell phone. The SSP may establish which sensors, software, parameters, etc. may be required for a given clinical trial, may include instructions for the patient to obtain a reading via one or more corresponding sensors, and/or may include various other types of data such as how sensors relate to each other, expected measure ranges of a sensor, etc. Based on the SSP, the patient may use sensors, e.g., a blood pressure cuff, a heartrate monitor, an accelerometer, a Global-Positioning System (GPS), etc., to collect one or more readings, e.g., blood pressure readings, as well as one or more other readings that provide context regarding the desired reading, such as patient heartrate, activity rate, location, motion, etc. The data from the readings may be collected via the user device.

Given the lack of supervision by a medical professional, the patient may struggle to collect the readings. For example, the patient may take measures in the wrong order, miss measures, use equipment incorrectly, etc. Based on the SSP, e.g., based on the parameters provided by the SSP for the given clinical trial, the integrity of the data collected by the user may be determined. For example, a patient may be instructed to obtain a measure using the blood-pressure cuff, and one or more additional sensors, e.g., the accelerometer, GPS, etc., may be used to provide context. For instance, such sensors may determine that, contrary to instructions, a patient was active or was not sitting while taking the blood pressure measure. The data may also be analyzed to verify provenance (e.g., to confirm patient identification). For example, the one or more readings may be compared to a biometric profile or fingerprint to verify that the subject of the readings is the correct patient. As a result of such analysis, the patient may be instructed to repeat the collection process, and/or the clinical trial may modify and/or contextualize the data for biasing factors.

While several of the examples above involve data readings of blood pressure for a clinical trial, it should be understood that techniques according to this disclosure may be adapted to any suitable data point(s) (e.g., blood test data (e.g., glucose, acidosis, etc.), pulse data, electroencephalogram data, electromusculogram data, etc.). It should also be understood that the examples above are illustrative only. The techniques and technologies of this disclosure may be adapted to any suitable activity. Presented below are various systems and methods of determining data provenance and/or data integrity.

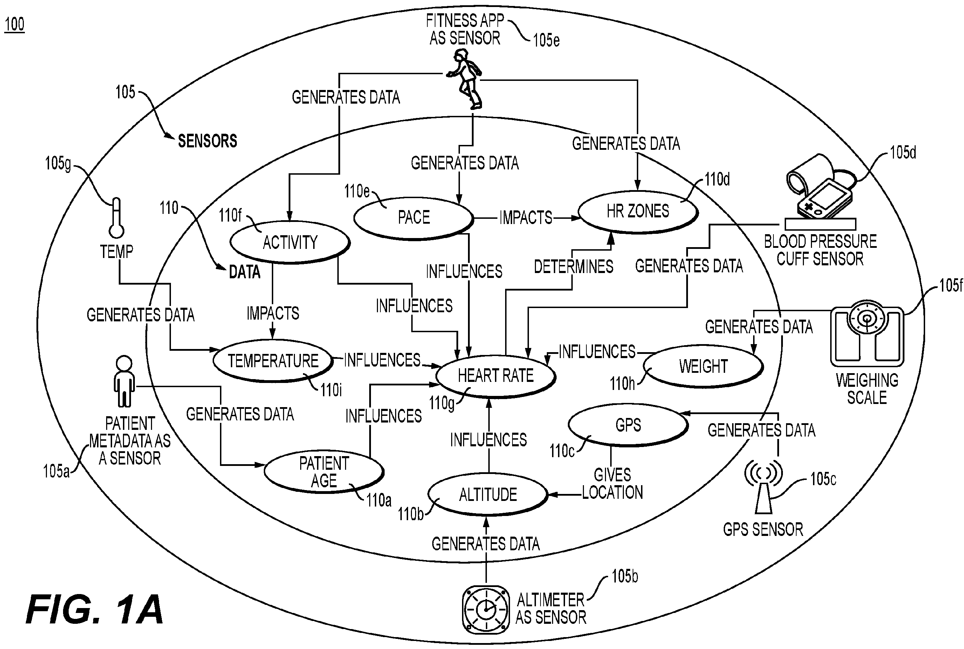

In some embodiments, as discussed in greater detail below, establishing and conducting a trial, e.g., a clinical trial, may require: developing a trial protocol (e.g., trial parameters, goals, etc.); requesting, screening, and accepting participants; sending the necessary sensors, devices, etc. to the participants; building and deploying a deployment package (e.g., an SSP) to a user device (e.g., a cell phone); obtaining readings from the participants; building and using patient-specific identification profiles (e.g., patient-specific biometric profiles); normalizing data (e.g., by contextualizing the data to environment, patient status, etc.); aggregating trial data from patients (e.g., to a centralized location for further analysis); analyzing the aggregated data (e.g., to establish relationships between data and/or sensors, as discussed in FIGs. 1A-1C, and/or to establish risk weights for future trials); etc.

Certain aspects of gathered data may be evaluated during and/or after conducting the trial, such as whether the data is valid, whether the data was taken using correct methodologies relative to the protocol, what the provenance of the data is, whether the data is corrupt in any discernable way (e.g., is the data an outlier?), whether the data comes from an expected patient (e.g., is the data from the study participant or an unrelated person?), and/or whether the absence of data represents a concern from a patient, cohort, and/or population. Such aspects may affect which data is processed, how the data is processed, and/or what relationships are developed between data and/or sensors.

In particular, the relationships between data and/or sensors may influence the sensors used, the data collected, the study conclusions, etc. For example, as further described below, the relationships between the data, between the sensors, or between the data and the sensors may determine what influences to consider when contextualizing the data. FIGs. 1A-1C depict exemplary relationships between the data, between the sensors, or between the data and the sensors that may influence determinations of protocol deviation, measurement validity, patient identification, measurement difficulty, overall risk, and/or data normalization.

FIG. 1A depicts an exemplary environment for collecting and/or influencing one or more readings, according to one or more embodiments. Environment 100 depicts that the one or more sensors 105 may be physical and/or virtual, and may be employed in a clinical trial to collect data 110 of a patient 102. For example, one or more sensors 105 may include a patient metadata sensor 105a, an altimeter sensor 105b, a GPS sensor 105c, a blood pressure sensor 105d, a fitness application sensor 105e, a weighing scale 105f, a temperature gauge (e.g., a thermometer) 105g, etc. Data 110 collected by the sensors may include patient age data 110a, altitude data 11 0b, GPS data 110c, heart rate zone data 1 10d, pace data 110e (e.g., running pace while using fitness application sensor 105e), activity data 110f (e.g., a user running while using fitness application sensor 105e), heart rate data 1 10g, weight data 1 10h, temperature data 1 10i, etc. Each of the one or more sensors 105 may be configured to perform multiple functions and may generate data across multiple relationships and/or interactions. For example, a cell phone may comprise patient metadata sensor 105a, altimeter sensor 105b, and GPS sensor 105c, and collect data 110 from each sensor. Data collected may influence, e.g., contextualize, other data. For example, altimeter sensor 105b may collect altitude data 1 10b. The altitude data 110b may be influenced by GPS data 110c to determine a user location, and the altitude data 110b may influence heart rate data 110g to determine heart rate zone data 110d. Any combination of one or more sensors 105 and/or data 110 may be used. The frequency of data collect may vary. For example, data collection may be a single collection in real time, an average of many collections over a period of time (e.g., 5 seconds), etc. Additional examples of sensors include but are not limited to HbAlc sensors, SPO$_2$ sensors (e.g., a pulse oximeter), electronic medical records, a patient diary or journal, electrocardiogram, electroencephalogram, force sensors, airflow sensors, pressure sensors, magnetometer, etc. Although FIG. 1A depicts several examples of sensors and certain exemplary combinations of sensors and data are discussed herein, it should be understood that any suitable sensor or combination of sensors may be used, e.g., based on the varying needs of a clinical trial. The metadata sensor 105a may be configured to electronically store patient-specific data in a manner which can be read by any suitable reader, e.g., in a standardized format. In one example, the metadata sensor 105a may include an RFID Identity card storing configured to be read to provide patient-specific data. Non-limiting examples of such data include phenotypic and genotypic data. Phenotypic data may include demographics and/or physical measurements, for example. As used herein, the term "virtual sensor" generally encompasses a sensor which is not necessarily physical in nature - but provides data nonetheless. One example would be if the patient data of the metadata sensor 105a is provided manually by the patient upon request, or if the physical metadata sensor is physically given to an administrator.

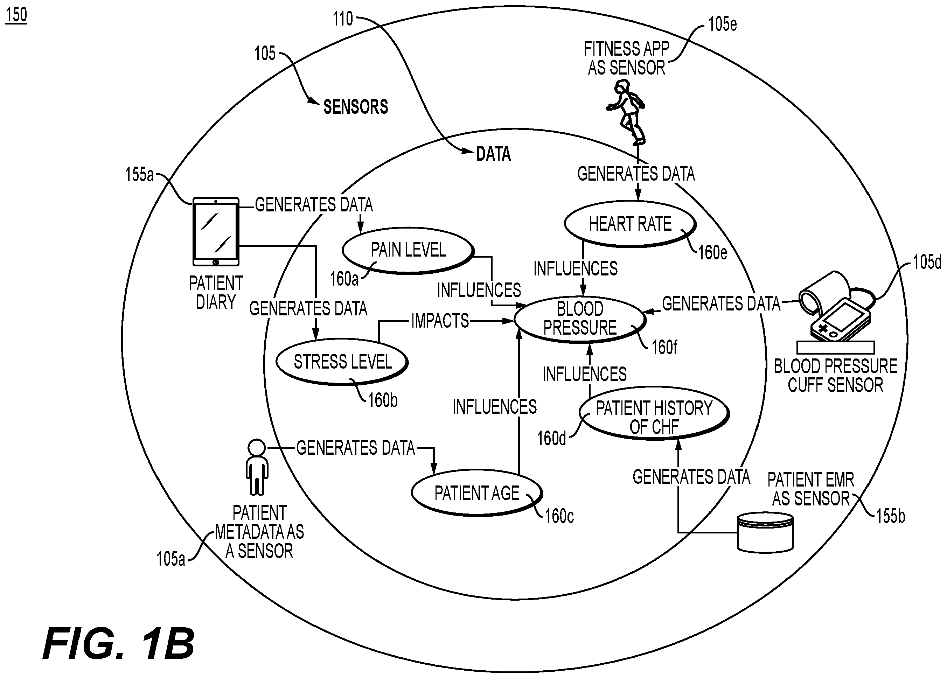

FIG. 1B depicts another exemplary environment for collecting and/or influencing one or more readings, according to one or more embodiments. Environment 150 of FIG. 1B depicts an exemplary relationship between one or more sensors 105 and data 110 to influence a determination of a user's blood pressure data 160f. However, it should be understood that other data may also be determined via the environment and/or other environments in other embodiments. In the exemplary environment 150 for determination of a user's blood pressure data 160f, one or more sensors 105 may include patient metadata sensor 105a, blood pressure sensor 105d, fitness application sensor 105e, a patient diary sensor 155a, an electronic medical records (EMR) sensor 155b, etc. Patient metadata sensor 105a may generate patient age data 160c. Blood pressure sensor 105d may generate blood pressure data 160f. Fitness application sensor 105e may generate heart rate data 160e. Patient diary sensor 155a may generate pain level data 160a and stress level data 160b, e.g., by parsing entries from the user into a patient diary (not shown). EMR sensor 155b may generate patient medical data, e.g., congestive heart failure (CHF) data 160d. Context for the blood pressure data 160f may be influenced to various degrees by one or more of patient age data 160c, heart rate data 160e, pain level data 160a, stress level data 160b, and/or CHF data 160d, etc., such as in the following example.

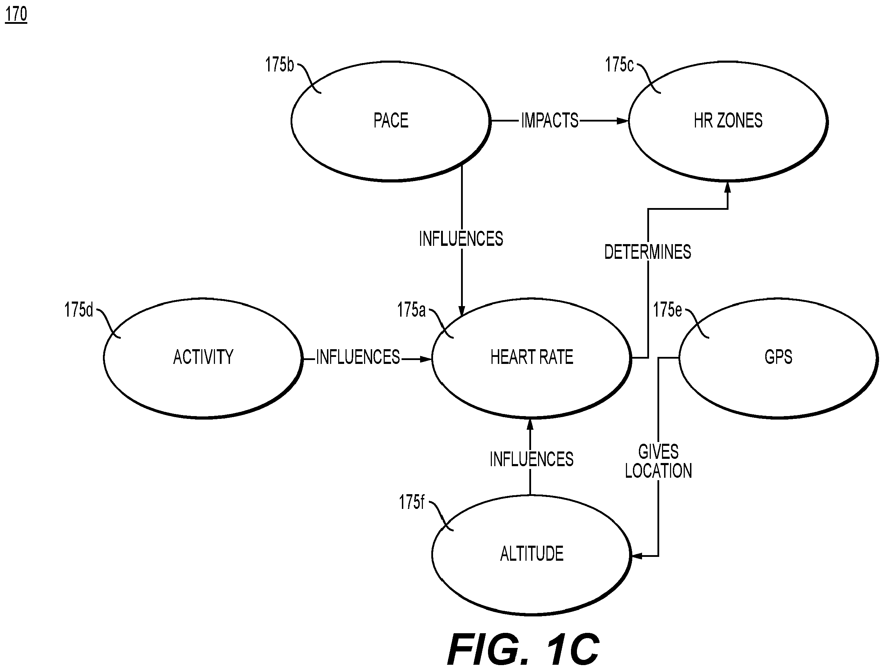

FIG. 1C depicts an exemplary environment for contextualizing data, according to one or more embodiments. Environment 170 of FIG. 1C depicts an exemplary relationship between various data points. As depicted in environment 170, the various data points may influence, determine, provide context, etc. on other data points. For example, GPS data 175e may provide location information for altitude data 175f, the contextualized altitude data 175f influencing the heart rate data 175a. Heart rate data 175a may be further influenced by activity data 175d and or pace data 175b, e.g., if patient 102 is exercising while the data is being collected. Heart rate data 175a and pace data 175b may influence the heart rate zones 175c, e.g., to determine the accuracy of a measured heart rate.

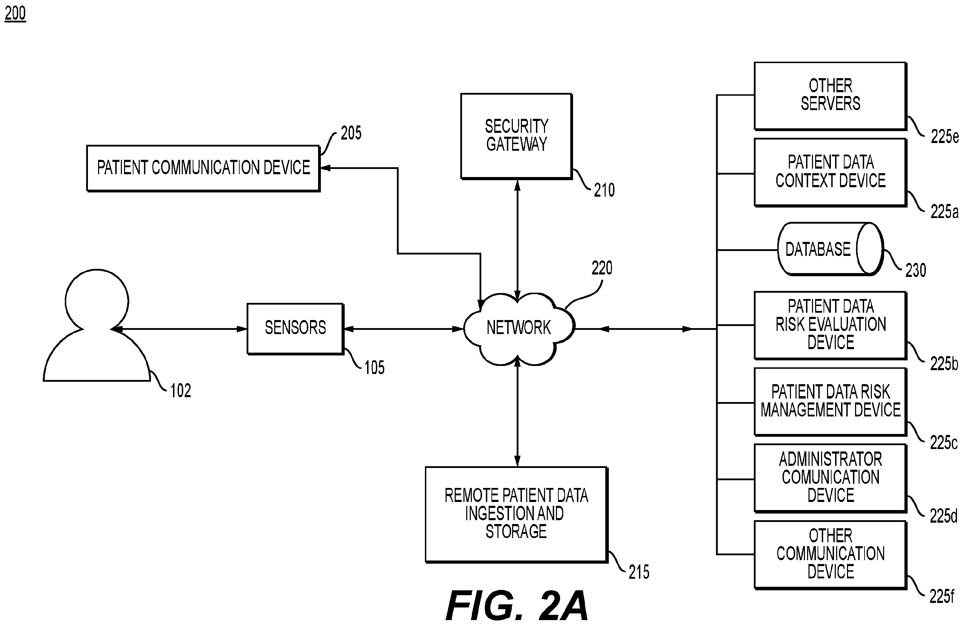

As depicted in FIGs. 1A, 1B, and 1C, one or more sensors 105 may interact to collect various forms of data 110. The data collected may influence other data, as described herein. In some embodiments, one or more sensors 105 may be operated by patient 102 from FIG. 2A, e.g., without the direction or supervision of a medical provider. For example, patient 102 may be participating in a clinical trial that requires regular collection of the pulse data of patient 102. Patient 102 may need to collect this pulse data throughout a day, making it impractical to have a physician collect the pulse data at each measurement time. However, patient 102 may collect data ineffectively and/or inappropriately, e.g., by not applying or using the one or more sensors 105 correctly, or in the presence of biasing factors. As such, medical providers and/or researchers may require methods to determine whether data 110 is reliable and/or accurate, e.g., a method for determining data provenance.

FIG. 2A depicts an exemplary environment for monitoring and determining data provenance and/or integrity, according to one or more embodiments. Environment 200 of FIG. 2A depicts one or more sensors 105, a patient communication device 205, a security gateway 210, a remote patient data ingestion and storage system 215, a network 220, a database 230, and various other systems. As described herein, one or more sensors 105 may provide data 110 to patient communication device 205, e.g., to remote patient data ingestion and storage system 215.

Patient communication device 205 may be any device configured to store, perform data integrity checks, measure risk, forward data for further analysis at a central processing server and application suite, etc. For example, patient communication device 205 may be a cell phone, a tablet, a computer, a smart watch, etc. Patient communication device 205 may use software previously downloaded, such as software downloaded by an SSP. As described in more detail herein, security gateway 210 may be any suitable gateway configured to securely distribute data, documents, etc., such as a base station, a router, an application, Secure File Transfer Protocol server, Application Programming Interface (API) gateway, etc. Remote patient data ingestion and storage system 215 may be configured to receive data from the one or more sensors 105 and/or transmit data, e.g., data received from the one or more sensors 105.

One or more of the components in FIG. 2A may communicate with each other and/or other systems, e.g., across network 220. In some embodiments, network 220 may connect one or more components of environment 200 via a wired connection, e.g., a USB connection between the one or more sensors 105 and patient communication device 205. In some embodiments, network 220 may connect one or more aspects of environment 200 via an electronic network connection, for example a wide area network (WAN), a local area network (LAN), personal area network (PAN), or the like. In some embodiments, the electronic network connection includes the internet, and information and data provided between various systems occurs online. "Online" may mean connecting to or accessing source data or information from a location remote from other devices or networks coupled to the Internet. Alternatively, "online" may refer to connecting or accessing an electronic network (wired or wireless) via a mobile communications network or device. The Internet is a worldwide system of computer networks-a network of networks in which a party at one computer or other device connected to the network may obtain information from any other computer and communicate with parties of other computers or devices. The most widely used part of the Internet is the World Wide Web (often-abbreviated "WWW" or called "the Web"). A "website page," a "portal," or the like generally encompasses a location, data store, or the like that is, for example, hosted and/or operated by a computer system so as to be accessible online, and that may include data configured to cause a program such as a web browser to perform operations such as send, receive, or process data, generate a visual display and/or an interactive interface, or the like. In any case, the connections within the environment 200 may be network, wired, any other suitable connection, or any combination thereof.

The various other systems may be configured to further process data 110, e.g., to determine the fitness-for-purpose of data 110. The various other systems may include a patient data context device 225a, a patient data risk evaluation device 225b, a patient data risk management device 225c, an administrator communication device 225d, other servers 225e, and/or other communication devices 225f, etc.

Patient data context device 225a may be configured to contextualize data 110, e.g., based on data source, data format, etc. For example, heart rate data 110g and pace data 110e may be received from blood pressure sensor 105d and fitness application sensor 105e, respectively. Patient data context device 225a may use pace data 110e to determine that heart rate data 110g is in a normal range for exercising, exceeds a normal range for exercising, etc. Patient data risk evaluation device 225b may be configured to examine data 110 to determine whether risks associated with data provenance are within predetermined risk thresholds, to perform various data integrity checks, e.g., patient identity verification, etc. As described in further detail below, the predetermined risk thresholds may be specific to each clinical trial.

Patient data context device 225a may include a design component and an execution component. The design component may be configured to determine relationships between the one or more sensors 105, between data 110, and/or between the one or more sensors 105 and data 110. Based on the determined relationships, patient data context device 225a may determine an algorithm design, discussed in further detail below, that may be configured to mathematically define the influences between the one or more sensors 105, e.g., between peripheral sensors (e.g., patient metadata sensor 105a, fitness application sensor 105e, etc.) and core sensors (e.g., blood pressure sensor 105d). The algorithm design may be configured into an executable series of steps similar to a workflow. It should also be understood that a sensor may operate as a peripheral sensor and/or as a core sensor in different scenarios, e.g., based on the needs of a clinical trial and/or how that sensor may relate to another sensor or sensors being used in the clinical trial. As noted above, the context device 225a may use determined sensor/data relationships to automatically configure algorithms. wherein an example, pace data may be related to the heart rate for a patient. There may be a simple linear relationship between pace and heart rate, which may be baselined to derive a correlation between the variables, e.g., either through a constant or through an algorithm (for situations where the relationship is not simple linear or exponential, for example). The baseline or relationship may be determined via observational studies. In the execution mode, that baselined relationship may be used to help determine whether there is a real problem indicated in the heart rate data. By the processor automatically analyzing the pace data and establishing the related heart rate from the observational study it can determine whether the heart rate data is higher than should be indicated.

As described in further detail below, patient data context device 225a may be configurable to combine data from multiple sources to normalize data, as discussed in further detail below. The configuration of patient data context device 225a may enable a trial designer to create semantically defined relationships between sensor data types, e.g., between GPS data 175e and altitude data 175f.

Patient data risk evaluation device 225b may be configured to evaluate the provenance and/or risk associated with data 110 and/or an overall risk associated with data 110. Exemplary methods of evaluating the provenance and/or risk are described in greater detail below.

As also described further below, patient data risk management device 225c may be configured to take action in response to certain determinations about data 110, e.g., in response to the provenance and/or risk evaluation by patient data risk evaluation device 225b. For example, if data 110 is determined to have a high risk value, patient data risk management device 225c may alert clinical trial staff, request patient 102 contact clinical trial staff, reject data 110 and/or request collection of data 110, etc.

Administrator communication device 225d may be the administrative console for the application and may be configured to facilitate a study team member configuring the sensors, the risk levels, weightings, and/or alerting workflows to be enacted as required for a specific study.

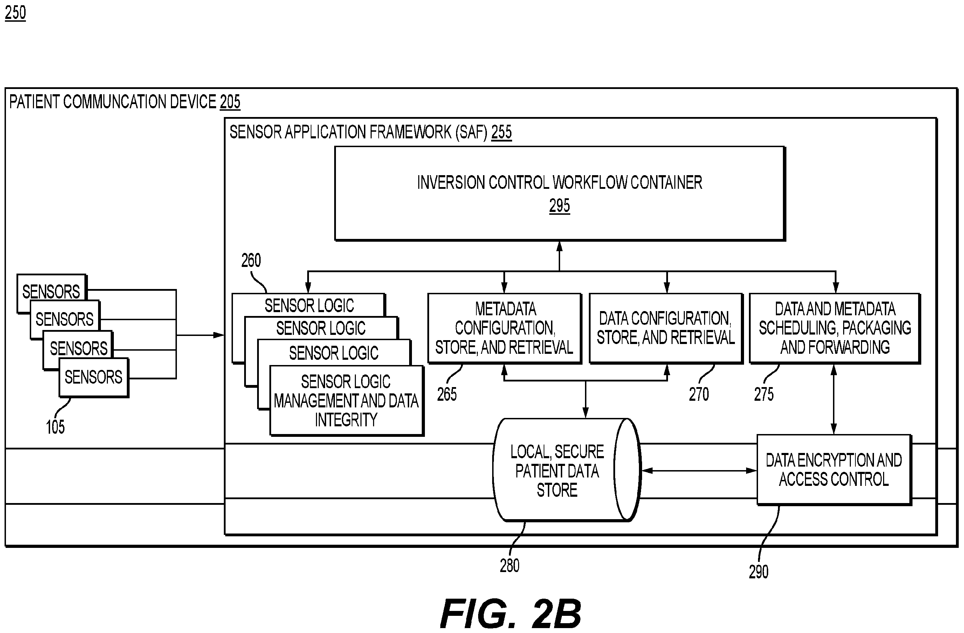

FIG. 2B depicts an exemplary Sensor Application Framework (SAF) 255, according to one or more embodiments. Environment 250 of FIG. 2B depicts that one or more sensors 105 may communicate with patient communication device 205 via SAF 255. SAF 255 may be installed centrally at a hub, in a distributed manner, e.g., on patient communication device 205, etc. As described in further detail below, SAF 255 may be configured to accept and/or install SSPs, which may be distributed as a part of a clinical trial, as described in further detail below.

As depicted in FIG. 2B, SAF 255 may comprise one or more sensor logic management and data integrity system 260, a metadata configuration, storage, and retrieval system 265, a data configuration, storage, and retrieval system 270, a data and metadata scheduling, packaging, and forwarding system 275, a local patient data store 280, a data encryption and access control system 290, an Inversion of Control (IoC) workflow container 295, etc. Regarding the encryption performed by the system 290, some specific examples include Elliptic Curve Cryptography (ECC) and Elliptical Curve Digital Signature Algorithm (ECDSA). Both are usable to validate the origin and integrity of messages.

The one or more sensor logic management and data integrity system 260 may include a SSP installation component, which may be configured to act as an install script and/or install one or more SSPs deployed to the SAF 255, as discussed further below. Operation of the one or more sensor logic management and data integrity system 260 may ensure that the metadata associated with the use of the sensor on the clinical trial is stored and executed as required during the clinical trial. For example, the one or more sensor logic management and data integrity system 260 may include a scheduler for a sensor that executes every 8 hours. The scheduler may analyse the metadata and/or data to be collected to determine whether the time, location, etc. match the data 110 that is collected. In some techniques, the one or more sensor logic management and data integrity system 260 may activate the one or more sensors 105 automatically, e.g., via an API.

Metadata configuration, storage, and retrieval system 265 may be configured to receive and/or store metadata, e.g., metadata received from patient metadata sensor 105a. Data configuration, storage, and retrieval system 270 may be configured to receive and/or store data, e.g., data received from blood pressure sensor 105d. Data and metadata scheduling, packaging, and forwarding system 275 may be configured to package and/or forward data and/or metadata based, for example, on a pre-determined schedule. For example, an SSP may be configured to instruct data and metadata scheduling, packaging, and forwarding system 275 to package and/or forward data and/or metadata (e.g., data 110) one a day, once a week, once a month, etc.

Data encryption and access control system 290 may be configured as an authorization and/or access control component for the data captured by the one or more sensors 105. Data encryption and access control system 290 may be configured to prevent unauthorized usage and/or access to data 110, e.g., via a role-based access control (RBAC) system capable of utilizing on-disk and inflight data encryption.

IoC workflow container 295 may be configured to enable SSPs to be self-contained and self-determine specific capabilities while using SAF 255 for common tasks, e.g., data configuration and basic metadata creation, storage and retrieval, forwarding of data, and/or other administrative features.

It should be noted that while these components are depicted separately, they may be distributed differently in various embodiments, e.g., metadata configuration, storage, and retrieval system 265 and data configuration, storage, and retrieval system 270 may be included as a single system.

As discussed herein, the data and one or more sensors may interact such that there may exist context between data, e.g., data 110, and/or the sensors, e.g., one or more sensors 105. Further, these interactions may provide context as to the manner in which patients collect samples, e.g., whether samples were collected incorrectly.

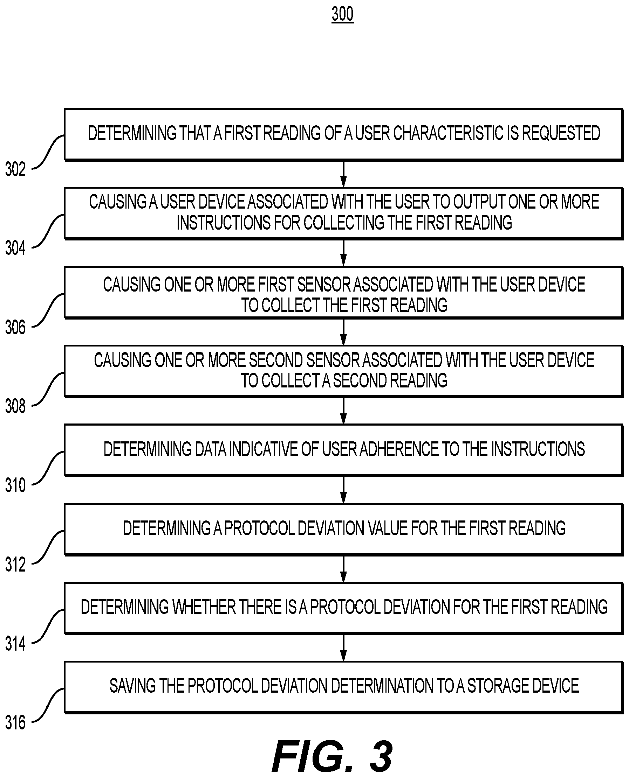

FIG. 3 depicts an exemplary method for determining data integrity and/or provenance, according to one or more embodiments. At step 302, a determination may be made that a first reading of a user characteristic may be requested. This determination may be based on a schedule instantiated during the installation of the SSP into SAF 255, e.g., as determined by the needs of the clinical trial in which the user is participating. Patient 102 may be prompted to collect a reading, the SAF 255 may be prompted to automatically take a reading from one or more sensors 105, or both may be prompted to occur.

At step 304, one or more instructions for collecting the first reading may be outputted to a patient communication device 205, e.g., a user's cell phone. The one or more instructions may be provided in response to a determination that patient 102 may be required to collect a reading. As described in further detail below, one or more instructions may be provided as part of the installation of the SSP to SAF 255.

The one or more instructions may provide step-by-step guidance on how to take the reading and/or may be based on the requirements of the clinical trial. For example, the instructions for a clinical trial on a sleep medication may vary from the instructions for a clinical trial on a heart medication. In another example, the instructions for a first heart medication clinical trial may vary from the instructions for a second heart medication clinical trial. In some embodiments, the one or more instructions may be configured to require the patient to verify each step, e.g., that each step is taken ahead of recording the reading. The one or more instructions and/or the verified steps may be locally saved, e.g., to local patient data store 280, prior to analysis and/or processing. Saving the instructions and/or verified steps to a local storage may bolster provenance and/or data integrity by ensuring that all attempts to take a reading are recorded.

At step 306, one or more first sensor associated with the user device may collect the first reading. At step 308, one or more second sensor associated with the user device may collect a second reading. For example, a first sensor (e.g., fitness application sensor 105e) may collect a first reading (e.g., heart rate data 160e), and a second sensor (e.g., EMR sensor 155b) may collect a second reading (e.g., historical patient heart rates).

In some embodiments, a sensor measurement data package may be generated. The sensor measurement data package may include the first reading, the second reading, and/or metadata associated with the first reading and/or second reading. The metadata may be metadata associated with a sensor-software combination that was in effect at the time of the first reading and/or the second reading. For example, if a first reading of stress level data 160b is collected from patient diary sensor 155a, metadata associated with patient diary sensor 155a and stress level data 160b at the time of collection may be generated. Collecting metadata may enable the full context of a reading to be reviewed during data analysis, such as for validation purposes.

In some embodiments, the data and metadata of the sensor measurement data package may be hashed, e.g., by data and metadata scheduling, packaging, and forwarding system 275. "Hashing" data refers to the process of passing data through a formula that produces a result, called a hash, e.g., by one-way encryption. As such, changes made to the data subsequent to hashing may be comparable to the hash value to determine type, timing, amount, etc. of the changes. The hashed data may be used in one or more subsequent steps of method 300. In some embodiments, the first reading, the second reading, the metadata associated with the first reading and/or the second reading, and/or the hashed data may be stored, e.g., in local patient data store 280 and/or database 230. The hash may, in one example, be 256 bit or 512 bit, e.g., depending on an amount of data to include in the hash to make it unique.

At step 310, data indicative of user adherence to the instructions (e.g., protocol deviation) may be determined. This determination may be based on one or more of the first reading, the second reading, or metadata associated with one or more of the first sensor or the second sensor, etc. The protocol deviation may be based on whether patient 102 selected the correct buttons, followed the required or necessary steps, provided proof that the steps were followed (e.g., photographs, videos, etc.), etc., as indicated by one or more readings from one or more sensors. In an example, fitness application sensor 105e may take the first reading of heart rate data 110g while GPS sensor 105c takes the second reading of GPS data 1 10c, showing that patient 102 was walking while the first reading was being collected. If the instructions indicated that patient 102 must be seated for 10 minutes before collecting the first reading, the first reading may be deemed to deviate from the trial protocol to a certain protocol deviation value.

At step 312, a protocol deviation value for the first reading may be determined. The protocol deviation value may be determined based on the second reading of step 308 and/or the data indicative of user adherence to the instructions of step 310. Continuing the prior example, if GPS sensor 105c determines that patient 102 was seated for only 7 minutes before collecting the first reading, the determined protocol deviation may be less than if GPS sensor 105c determines that patient 102 was seated for only 1 minute before collecting the first reading.

In some embodiments, the protocol deviation value may be a point system, such that greater numbers and/or amounts of variations/deviations may increase or decrease the protocol deviation value. Further continuing the prior example, a determination that patient 102 was seated for only 7 minutes before collecting the first reading may correspond to 3 points-one point for each minute patient 102 failed to conform to the instructions-whereas a determination that patient 102 was seated for only 1 minute before collecting the first reading may correspond to 9 points. If further deviations are determined, such as missing readings, improperly applied sensors, etc., these deviations may have corresponding points that may affect the protocol deviation value for a given reading. It should be noted that a point system is an exemplary method for determining the protocol deviation value, and any suitable scoring system may be used.

At step 314, whether there is a protocol deviation may be determined. The presence of a protocol deviation may be determined based on a comparison of the determined protocol deviation value with a deviation threshold value.

In some embodiments, if the protocol deviation value exceeds the deviation threshold value, a risk indicator, e.g., a protocol deviation risk indicator, may be appended to the reading data. The protocol deviation risk indicator may, for example, inform clinical trial managers of the protocol deviation associated with a respective reading.

At step 316, the protocol deviation determination may be saved to a storage device, e.g., local patient data store 280. In some embodiments, the protocol deviation may be determined by each respective sensor and/or protocol deviation software that may be customized to each respective sensor. The protocol deviation software may be configured ahead of the one or more sensors 105 and/or SAF deployment.

In some embodiments, additional data risk dimensions may be determined. For example, one or more of measurement validity, patient identification (e.g., biometric), a difficulty score, and/or an overall risk may be generated. The measurement validity, or reading validity, may be used to determine whether the reading, e.g., the first reading, was within an expected range. The expected range may be based on patient 102, the clinical trial, the sensor used, etc. For example, the measurement validity may be used to determine whether the first reading exceeds the expected range due to an error relative to cohort averages or trends specific to patient 105.

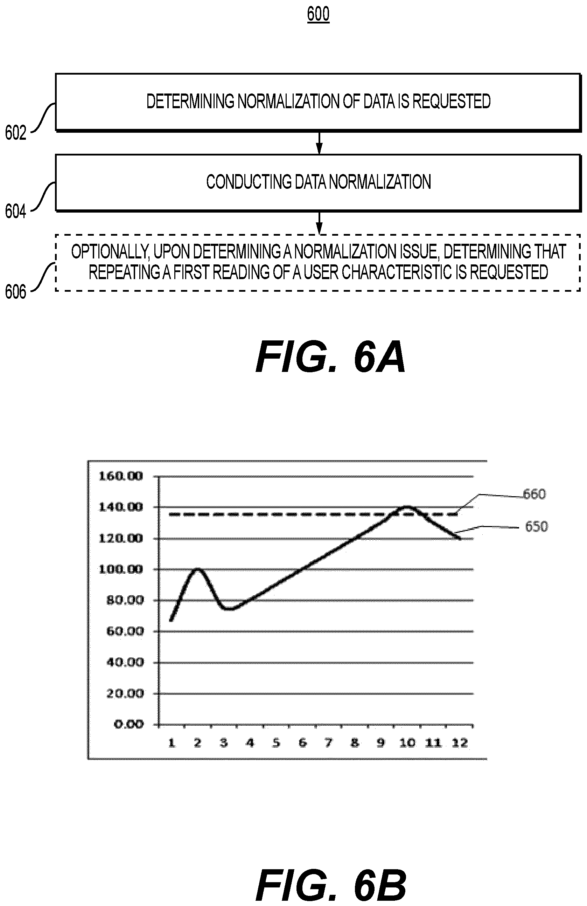

The measurement validity may be determined by comparing a validity measure to a validity measure threshold. Whether the measurement validity exceeds an expected range may be determined by generating a validity range specific to patient 102. The validity range may be based on one or more of the first reading, the second reading, or metadata associated with one or more of the first sensor and/or the second sensor. The first reading may be compared to the validity range to determine the validity measure. The validity measure may be compared to the validity measure threshold to determine the measurement validity. For example, a first reading may be compared to a pre-determined heart rate validity range for patient 102, as discussed in further detail below with regard to FIG. 6. Fig. 6B is a representative plot, the vertical axis being a parameter for readings, in this case average heart rate, and the horizontal axis being time of the readings. In FIG. 6B, an average heart rate 650 is plotted against an upper threshold 660 for heart rate.

The patient identification may be used to determine whether the user collecting the reading is actually patient 102 or another user, e.g., an unauthorized user. In some embodiments, biometrics (e.g., biometrics associated with the first reading or the second reading) may be used to determine patient identification. Biometrics may be one or more of multi-factor authentication, biometric fingerprints (e.g., physical fingerprint, handwriting analysis, patient-specific electroencephalogram readings, iris and/or retina scans, hand geometry, voice and/or facial recognition, etc.), pre-established credentials (e.g., a username and password), knowledge-based identification (e.g., patient-specific questions), etc. In some embodiments, the biometric data may be associated with, e.g., appended to, the first reading.

The patient identification (e.g., a user biometric identification) may be determined in response to the determination that a first reading of a user is requested (e.g., in response to step 302). In some embodiments, a biometric measure may be compared to a biometric threshold to determine a user biometric identification. The biometric measure may be determined by comparing a biometric reading with a patient-specific biometric profile. The biometric reading may be generated based on on one or more of the first reading, the second reading, and/or metadata associated with one or more sensor 105. For example, patient 102 may enter a fingerprint, which may be compared to prior fingerprint entries of patient 102. In some embodiments, this comparison is based on a threshold. In some embodiments, the patient identification may be dynamic, e.g., may change and/or update over time based on changes in a user's characteristics. For example, facial recognition software may automatically or manually update to recognize patient 102 before, during, and after weight loss.

In some embodiments, a machine learning model may be used to determine the patient identification. For example, a machine learning model may be used to generate patient-specific biometric profile from verified and/or validated patient data over a given amount of time. The machine learning techniques that may be used include Classification or Outlier Detection.

Classification is a supervised learning approach in which the computer program learns from the data given to it and makes new observations or classifications. Classification may be completed via any suitable classification algorithm, including but not limited to Logistic Regression, Naive Bayes, Stochastic Gradient Descent, K-Nearest Neighbors, Decision Tree, Random Forest, Artificial Neural Network, Support Vector Machine, etc. The classification algorithm may be binary or multi-class. For example, a classification algorithm may determine whether a patient identification data point is a "match" or "not a match."

Outlier detection is a machine learning approach in which an object that deviates significantly from the rest of the objects is analyzed to determine the significance and/or relevance of the deviation. Outlier detection may be completed via any suitable outlier detection algorithm, including but not limited to box plotting, interquartile ranging, z-scoring, multivariate distancing, K-Means clustering, etc. For example, an outlier detection algorithm may determine a fingerprint received is an outlier and may therefore reject the patient identification attempt.

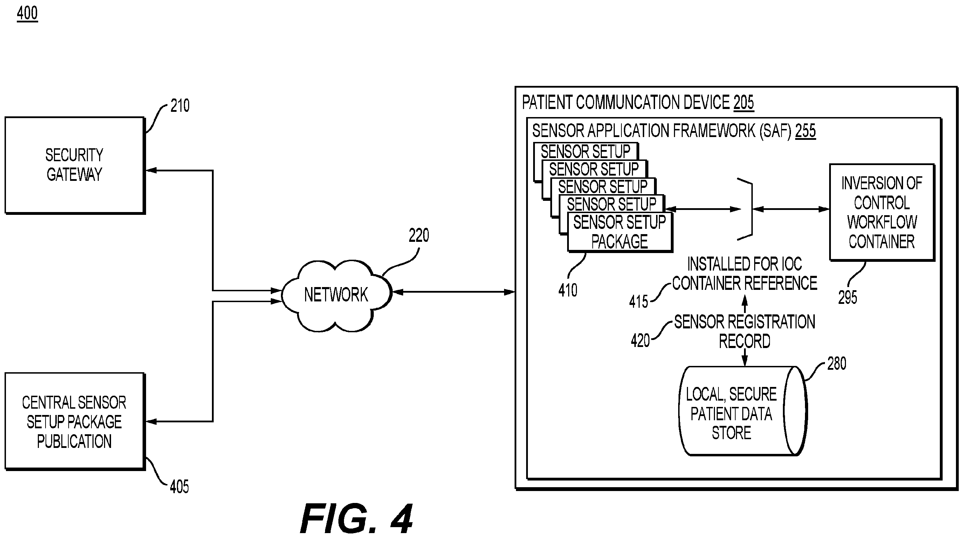

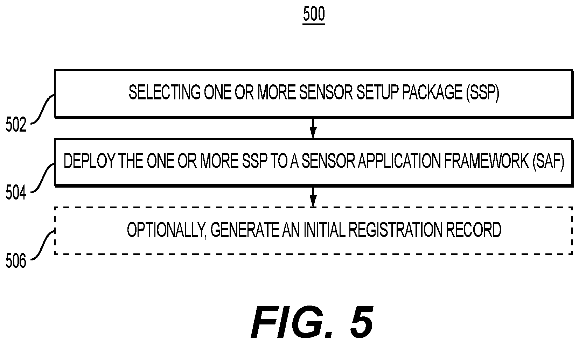

The machine learning model may be trained using any suitable method or data. The machine learning model, as disclosed herein, may be trained using environment 100 of FIG. 1A, environment 150 of FIG. 1B, environment 170 of FIG. 1C, environment 200 of FIG. 2A, environment 250 of FIG. 2B, method 300 of FIG. 3, environment 400 of FIG. 4, method 500 of FIG. 5, and/or method 600 of FIG. 6. The machine learning model may be trained using training data, which may include one or more of data 110, metadata associated with data 110, other data relevant to a given clinical trial, etc.

The machine learning networks and/or models may include, but are not limited to, a deep learning network such as Deep Neural Networks (DNN), Convolutional Neural Networks (CNN), Fully Convolutional Networks (FCN), and/or Recurrent Neural Networks (RCN), probabilistic models such as Bayesian Networks and Graphical Models, and/or discriminative models such as Decision Forests and maximum margin methods, or the like.

The difficulty score may be a measurement difficulty, or how difficult collecting the measurement may be for a user. The difficulty score may be determined for a reading based on historical information, e.g., historical information associated with the first reading from the user, from other users, or both. In some embodiments, the historical information may include other data indicative of user adherence (protocol deviation, measurement validity, patient identification, etc.), the number of attempts needed for patient 102 to record the first reading, etc. In some embodiments, the difficulty score may be appended to the first reading data, e.g., as a difficulty score risk indicator.

In some embodiments, the overall risk may be determined based on one or more configurable factors. The overall risk may be determined by weighing the one or more configurable factors, e.g., by weighing the relative importance of the one or more configurable factors. The importance may be determined by the nature of the configurable factor, the trial protocol, etc. The one or more configurable factors may include the protocol deviation (e.g., patient 102 adherence to the instructions provided in step 304), the patient identity (e.g., biometric evidence that patient 102 collected the reading on themselves), the measurement validity (e.g., whether the reading is within the expected range), the difficulty score (e.g., how many times patient 102 attempted to collect a reading compared to a standard value), etc.

In some embodiments, the overall risk may be determined using an algorithm, such as the following algorithm.

$$overallRisk(measurement) = \sum_{i=1}^{i=n} riskDimension(i) * riskWeighting(i)$$

The value of "n" may be the number of risk dimensions determined by the clinical trial design and the value of "i" may be the current dimension of risk under consideration. The algorithm may take into account the weighing of one or more configurable factors, such as the relative importance of each configurable factor. As such, the algorithm may provide an overall risk determination to a patient-specific and/or trial-specific level.

The factors used to determine the one or more data risk dimensions may vary between SSPs, clinical trials, devices, SAFs, etc. For example, a first clinical trial may determine protocol deviation and patient identity, whereas a second clinical trial may determine measurement validity and measurement difficulty. In some embodiments, metadata regarding data risk dimensions, measurement difficulty, or the like may be aggregated for various types of sensors, such that such metadata may thereafter be applied to future trials that include one or more similar sensor.

In some embodiments, the SSP(s) for a given clinical trial may determine SAF functionality. For example, a first SSP may provide logic for determining data provenance of electroencephalogram sensors, and a second SSP may provide logic for determining data provenance of metadata sensors. In this way, an SSP may be configured to determine, e.g., via IoC workflow container 295, which sensors, sensor profiles, data, instructions, software, etc. may be necessary for a given clinical trial. The security gateway 210 may be configured to perform one or more functions to ensure that the data traversing the system maintains its integrity, e.g., by ensuring traceability from the patient through its final delivery. This may include encryption, and/or other algorithms that may be in place to ensure the identity of the patient matches the identity associated with the readings which may be coming from the patient (outlier detection and data classification are two possible means by which this could be achieved).

FIG. 4 depicts an exemplary schematic for SSP deployment, according to one or more embodiments. Environment 400 of FIG. 4 depicts a security gateway 210, a network 220, a central SSP publication device 405, and an SAF 255 of a patient communication device 205. The central SSP publication device 405 may publish the various SSPs available for deployment to SAF 255. In some embodiments, blockchain may be used to generate and deploy SSPs, e.g., by IoC workflow container 295 across a peer-to-peer network. For example, generation and deployment of the SSPs may be tracked via blockchain to track the provenance of the SSPs, which may ensure that the SSPs have not been tampered with prior to use by patient 102.

SAF 255 may include one or more SSP 410, IoC workflow container 295, a sensor registration record 420, and/or local patient data store 280. The one or more SSP 410 may be downloaded to a location accessible by the SAF, e.g., central SSP publication device 405. The one or more SSP 410 may be installed for IoC workflow container reference (step 415).

Sensor registration record 420 may be generated by the SAF. Sensor registration record 420 may include one or more of patient-readable instructions, configuration data, metadata, etc. The patient-readable instructions may include training on the setup and use of the sensor provided for the clinical trial, e.g., a blood pressure sensor. The configuration data may include data for determining the scheduling, sensor software API, and other logistical information associated with the sensor and the data to be gathered.

The metadata may describe the data to be collected from the sensor, the context of the data to be taken, the risk factors associated with the one or more sensors 105, etc. The metadata describing the data to be collected from the sensor may include units of measure (e.g., mmHg for blood pressure); minimums, maximums, expected value ranges, determination of measurement variance, thresholds, etc. (e.g., patient-specific blood pressure ranges); device descriptors (e.g., serial number, model number, date of manufacturer, manufacturer, compliance certificates, security certificates, etc.); software descriptors (e.g., version, build numbers, date/time of creation, date/time of installation, etc.); etc. The metadata describing the context of the data to be taken may include methodologies, validation steps prior to submission, etc. The metadata describing the risk factors associated with the one or more sensors 105 may include metadata on the importance of the risk factors associated with the sensor and the data to be gathered by the patient.

By deploying SSP(s) in the manner discussed above, various aspects of the operation of the clinical trial may be automated. For example, since the SSP(s) for a clinical trial may include patient-readable instructions, the deployment of a set of SSPs for a clinical trial may result in an automatic assembly of instructions for the use of the various sensors at play in the clinical trial. In another example, since the SSP(s) may include data regarding relationships between sensors, the set of SSPs may result in automatic determination of the context between the various sensors, the sensors usable for and protocol for evaluating patient compliance with instructions, expected ranges for sensor readings, etc. In other words, in some embodiments, the protocol for a clinical trial, e.g., readings desired and sensors used, may be usable to automate development of a custom software package that is tailored to the clinical trial and that provides patient instruction, determines data provenance and/or integrity, and provides context for measured data.

FIG. 5 depicts an exemplary method for deploying an SSP, according to one or more embodiments. At step 502, one or more SSP 410 may be selected. The one or more SSPs 410 may be selected based on the needs of the clinical trial prior to distribution, acceptance, and installation of the one or more SSPs in the SAF. The needs of the clinical trial may be determined by the SAF, e.g., by the IoC workflow container 295. For example, the requisite number, type, specific configuration, and logic of the one or more SSP 410 may be determined prior to distribution to the SAF by the IoC workflow container 295. The IOC workflow container 295 may be configured to function similarly or differently across one or more sets of SSPs, one or more trials, etc., such that a single IOC workflow container 295 may be used in multiple clinical trials at a time and for different functions. Regarding step 502, in one example, selection may be performed via a graphical user interface in which a menu of available components are selected from, e.g.: multi-media video files, pdf documents, installable software components (for example the equivalent of jar files, dll files, or executables), driven by the requirements of the trial in terms of what is available and "packagable" together, e.g., as a "zip file".

At step 504, the one or more SSP 410 may be deployed to the SAF 255. The one or more SSP 410 may be configured to self-execute, e.g., self-install on the SAF 255.The one or more SSP 410 may establish which sensors, software, parameters, etc. may be required for a given clinical trial. The publication and/or deployment of the one or more SSP 410 may be via a secure mechanism, e.g., security gateway 210. In some embodiments, security gateway 210 may perform functions as described in European Patent No. 3,620,937 A1, which is incorporated herein by reference.

Optionally, at step 506, sensor registration record 420 may be generated. Sensor registration record 420 may include data as described in herein. Sensor registration record 420 may be stored in a data store, e.g., in local patient data store 280. In some techniques, sensor registration record 420 may be automatically stored in response to installation, e.g., self-installation, or the one or more SSP 410.

In some embodiments, the data may be normalized, e.g., contextualized. Even if patients correctly or mostly correctly collect measurements, variations external to collection, e.g., the patient's environment, may affect the readings. For example, a properly collected heart rate reading for a given patient may be significantly different at sea level as opposed to at the top of Mount Everest due to the effect of altitude on the patient's heart (e.g., higher altitude increases the workload on the heart, requiring a higher average heart rate as compared to the sea level workload). Therefore, even if the patient properly collects the heart rate measure in both locations, the measure may be vastly different. FIG. 6 describes an exemplary method for contextualizing readings, e.g., based on altitude. This normalization process may reduce erroneous determinations of inaccuracy and/or unreliability. in an example of normalized data, a baseline measurement is taken for a cohort. Then, measurements are taken from the individual and expressed as a percentage of the baseline measurement. Normalization may also take the form of a number relative to an average, or where multiple measurements together form an observation, whereby that observation may be used as a relative ranking of patient risk, health, or other factors.

FIG. 6A depicts an exemplary method for normalizing data, e.g., contextualizing data, according to one or more embodiments. At step 602, a request for data normalization may be determined. At step 604, data normalization may be conducted, e.g., by patient data context device 225a. The risk dimensions discussed herein (e.g., protocol deviation, measurement validity, patient identification, difficulty score, and/or overall risk) and/or other data may be normalized using custom, extendable computational modules and may be used as input to an overall risk assessment that may be associated with the reading. Metadata may be added to raw data, e.g., data collected from one or more sensors 105, along with the required local analysis, e.g., in a form that is useful for the clinical trial, which may be added to the provenance of the initial raw data set. The metadata may provide contextualization, e.g., by providing information related to the time a reading was taken.

For example, a trial designer may deploy an algorithm that may normalize heart rate data 175a based on patient age data 160c, pace data 175b, altitude data 175f, and/or GPS data 175e as shown in the example below.

> If: patient age between 40 and 50 years
>
> If: pace exceeds 10 kilometers per hour
>
> If: altitude and/or GPS indicate hilly terrain
>
> If: heart rate (HR) exceeds 130 beats per minute
>
> NormailisedHR = HR / 2
>
> Else
>
> NormailisedHR = HR /1.33
>
> If: heart rate does not exceed 130 beats per minute
>
> NormailisedHR = HR / 1.5
>
> Else
>
> NormailisedHR = HR /1.2

Alternatively or in addition, normalization may modify thresholds associated with the first readings and/or the second readings as shown in the example below.

> If: patient age between 40 and 50 years
>
> If: pace exceeds 10 kilometers per hour
>
> If: altitude and/or GPS indicate hilly terrain
>
> If: heart rate exceeds 130 beats per minute
>
> HeartRate.UpperThreshold = HeartRate.UpperThreshold * 1.25
>
> HeartRate.LowerThreshold = HeartRate.LowerThreshold # Unchanged
>
> Else
>
> HeartRate.UpperThreshold = HeartRate.UpperThreshold * 1.15
>
> HeartRate.LowerThreshold = HeartRate.LowerThreshold # Unchanged

As such, patient data context device 225a may provide contextualization of the reading data. Normalization may enable modification of the risk dimensions and thresholds, which may be dependent on the context within which sensor readings are taken and which may change over time. For example, for a patient that often travels to various altitudes, the relationship between data 110, e.g., heart rate data 1 10g, and altimeter sensor 105b may be heavily weighted in the normalization process.

In some embodiments, the normalization process, e.g., normalization codes, may be predetermined, e.g., by the one or more SSP 410, and/or determined by the system, e.g., the systems of FIGs. 2A-2B. In some embodiments, a machine learning model may be used to determine the relationships and the relative importance of those relationships, e.g., based on a given trial protocol, the habits of patient 102, etc. For example, for a patient that goes for a run every evening, a machine learning model may contextualize an elevated evening heart rate measure based on other sensors detecting exercise, and may prioritize contextualizing data from those sensors based on the weighted impact on the readings. The machine learning model may be trained using methods described herein.

At step 606, upon determining a normalization issue, e.g., based on a normalization threshold, repetition of a first reading of a user characteristic may be optionally requested. If one or more problems are discovered with the measurement or the metadata, or if the risk profile is determined to cross a threshold of acceptance by the clinical trial protocol, patient 102 may be prompted to attempt to retake the measurement. For example, if heart rate data 175a are determined to fall outside of heart rate zone 175c for a given patient, that patient may be prompted to retake the measurement of heart rate data 175a. Patient 102 may be requested to repeat the readings until patient 102 either tries unsuccessfully for a specific number of attempts or takes the measurement successfully. The reading data, collection data, metadata, etc. may be stored, e.g., to local patient data store 280.

At predefined intervals, data (e.g., reading data, normalized data, further normalized data, etc.) may be published to one or more systems, e.g., administrator communication device 225d, for further interpretation and analysis. The data may be published in response to a data provenance determination, SSP deployment, normalization, further normalization, etc. In some embodiments, missing or improperly collected readings may be published as an empty dataset, and may include an error or warning code. For example, if patient 102 fails to collect heart rate data 160e within an allowed number of attempts, the readings may be rejected and then published as an empty set with a warning that patient 102 may have exceeded the allotted number of attempts. In some embodiments, an acknowledgement or patient engagement notice may be prompted in response to a determination of missing or improperly collected readings. For example, if an empty set is published three times in a row, patient 102 may receive a reminder ping, e.g., at patient communication device 205.

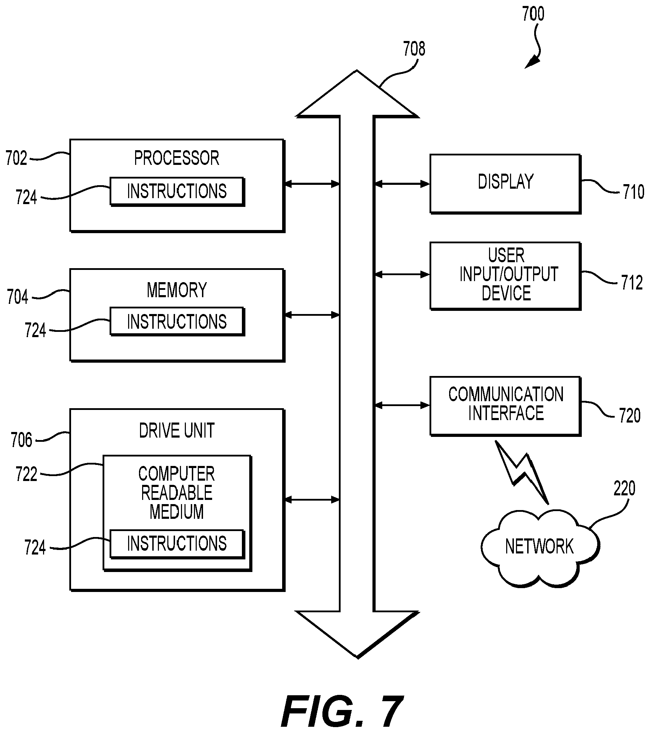

FIG. 7 depicts a simplified functional block diagram of a computer, according to one or more embodiments. FIG. 5 depicts a simplified functional block diagram of a computer 700 that may be configured as a device for executing the methods of FIGs. 3, 5, and 6 according to exemplary embodiments of the present disclosure. One or more of a processor 702, a memory 704, a drive unit 706, an internal communication bus 708, a display 710, a under input/output ports 712, a communication interface 720, a computer readable medium 722, instructions 724, and a network 220 may communicate by any suitable means. For example, computer 700 may be configured as the one or more sensors 105, patient communication device 205, and/or another system according to exemplary embodiments of this disclosure. In various embodiments, any of the systems herein may be a computer 700 including, for example, data communication interface 720 for packet data communication. Computer 700 also may include a central processing unit (CPU) 702, in the form of one or more processors, for executing program instructions. Computer 700 may include internal communication bus 708, and storage unit 706 (such as Read-Only Memory (ROM), Hard Disk Drive (HDD), Solid-State Drive (SSD), etc.) that may store data on computer readable medium 722, although computer 700 may receive programming and data via network communications. Computer 700 may also have memory 704 (such as Random-Access Memory (RAM)) storing instructions 724 for executing techniques presented herein, although instructions 724 may be stored temporarily or permanently within other modules of computer 700 (e.g., processor 702 and/or computer readable medium 722). Computer 700 also may include input and output ports 712 and/or display 710 to connect with input and output devices such as keyboards, mice, touchscreens, monitors, displays, etc. The various system functions may be implemented in a distributed fashion on a number of similar platforms, to distribute the processing load. Alternatively, the systems may be implemented by appropriate programming of one computer hardware platform.

Program aspects of the technology may be thought of as "products" or "articles of manufacture" typically in the form of executable code and/or associated data that is carried on or embodied in a type of machine-readable medium. "Storage" type media include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer of the mobile communication network into the computer platform of a server and/or from a server to the mobile device. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

While the disclosed methods, devices, and systems are described with exemplary reference to transmitting data, it should be appreciated that the disclosed embodiments may be applicable to any environment, such as a desktop or laptop computer, an automobile entertainment system, a home entertainment system, medical equipment, etc. Also, the disclosed embodiments may be applicable to any type of Internet protocol.

It should be appreciated that in the above description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments may be used in any combination.

Thus, while certain embodiments have been described, those skilled in the art will recognize that other and further modifications may be made thereto without departing from the spirit of the invention, and it is intended to claim all such changes and modifications as falling within the scope of the invention. For example, functionality may be added or deleted from the block diagrams and operations may be interchanged among functional blocks. Steps may be added or deleted to methods described within the scope of the present invention.

The above disclosed subject matter is to be considered illustrative, and not restrictive, and the appended claims are intended to cover all such modifications, enhancements, and other implementations, which fall within the true spirit and scope of the present disclosure. Thus, to the maximum extent allowed by law, the scope of the present disclosure is to be determined by the broadest permissible interpretation of the following claims and their equivalents, and shall not be restricted or limited by the foregoing detailed description. While various implementations of the disclosure have been described, it will be apparent to those of ordinary skill in the art that many more implementations are possible within the scope of the disclosure. Accordingly, the disclosure is not to be restricted except in light of the attached claims and their equivalents.

## Claims

1. A computer-implemented method for determining data integrity, the method comprising:

   determining (302), via a processor (702), that a first reading of a user characteristic is requested;
   causing (304) a user device (205) associated with the user to output one or more instructions for collecting the first reading, the instructions based on a predetermined protocol;
   causing (306) one or more first sensor (105) associated with the user device to collect the first reading;
   causing (308) one or more second sensor (105) associated with the user device to collect a second reading;
   determining (310), via the processor, based on one or more of the first reading, the second reading, or metadata associated with one or more of the first sensor or the second sensor, data indicative of user adherence to the instructions;
   determining (312), via the processor, a protocol deviation value for the first reading based on the second reading and the data indicative of user adherence to the instructions;
   determining (314), via the processor, whether there is a protocol deviation for the first reading based on a comparison of the determined protocol deviation value with a deviation threshold value; and
   saving (316) the protocol deviation determination to a storage device (280).

2. The method of claim 1, wherein:

   the predetermined protocol further includes a schedule for requesting the first reading; and
   the determining that a first reading of a user characteristic is requested is based on the predetermined protocol.

3. The method of claim 1 or claim 2, further comprising:
   hashing the metadata (265) to at least one of the first reading or the instructions.

4. The method of any preceding claim wherein, the processor is configured to, upon determining that there is no protocol deviation, append (225b, 225c) a risk indicator to the first reading, the risk indicator based on the determined protocol deviation value.

5. The method of any preceding claim, wherein the processor is configured to, upon determining that there is a protocol deviation, cause the one or more first sensor (105) associated with the user device to collect a further first reading.

6. The method of any preceding claim, further comprising:
determining (260) a measurement validity of the first reading via one or more of the first sensor or the second sensor.

7. The method of claim 6, further comprising:

generating (260, 265) a validity range specific to the user based on one or more of the first reading, the second reading, or metadata associated with one or more of the first sensor or the second sensor; and
determining a validity measure based on a comparison of the first reading with the validity range specific to the user,
wherein determining the measurement validity includes determining whether the validity measure exceeds a validity measure threshold.

8. The method of any preceding claim, further comprising:
determining (302) a user biometric identification in response to determining that the first reading of a user is requested.

9. The method of claim 8, further comprising:

generating (302) a biometric reading based on one or more of the first reading, the second reading, or metadata associated with one or more of the first sensor or the second sensor; and
determining a biometric measure based on a comparison of the biometric reading with a patient-specific biometric profile,
wherein determining (314) the user biometric identification includes comparing the biometric measure with a biometric threshold.

10. The method of any preceding claim, further comprising:

obtaining (602) a difficulty score for the first reading based on historical information associated with the first reading, the historical information including one or more of historical information regarding the data indicative of user adherence to the instructions or historical information regarding a number of attempts needed to record the first reading; and
appending a further risk indicator to the first reading, the risk indicator based on the obtained difficulty score.

11. The method of any preceding claim, further comprising:
determining an overall risk associated with the first reading, the overall risk determined based on one or more factors with associated weightings.

12. The method of any preceding claim further comprising:

determining (302), via the processor, a user biometric identification based on a comparison of a biometric measure with a biometric threshold, wherein the biometric measure is determined based on a comparison of a biometric reading with a patient-specific biometric profile;
determining (225b, 225c), via the processor, a measurement validity of the first reading based whether a validity measure exceeds a validity measure threshold, wherein the validity measure is determined based on a comparison of the first reading with a validity range specific to the user;
obtaining a difficulty score for the first reading based on historical information associated with the first reading, the historical information including one or more of historical information regarding the data indicative of user adherence to the instructions or historical information regarding a number of attempts needed to record the first reading;
appending (225b) a risk indicator for each of the user biometric identification, the protocol deviation value, the measurement validity, and the difficulty score to the first reading; and
determining (225b) an overall risk associated with the first reading, the overall risk determined based on the risk indicators for each of the user biometric identification, the protocol deviation value, the measurement validity, and the difficulty score.

13. A system, the system comprising:

at least one memory (230) storing instructions; and
at least one processor (225) configured to execute the instructions to perform operations for determining data integrity, the operations including:

determine (302) that a first reading of a user characteristic is requested;
cause (304) a user device associated with the user to output one or more instructions for collecting the first reading, the instructions based on a predetermined protocol;
cause (306) one or more first sensor associated with the user device to collect the first reading;
cause (308) one or more second sensor associated with the user device to collect a second reading;
determine (310) based on one or more of the first reading, the second reading, or metadata associated with one or more of the first sensor or the second sensor, data indicative of user adherence to the instructions;
determine (312) a protocol deviation value for the first reading based on the second reading and the data indicative of user adherence to the instructions;
determine (314) whether there is a protocol deviation for the first reading based on a comparison of the determined protocol deviation value with a deviation threshold value; and
save (316) the determined protocol deviation to a storage device.

14. The system of claim 13, further comprising:
hashing the metadata to at least one of the first reading or the instructions.

15. The system of claim 13 or claim 14, wherein, the at least one processor (225c) is configured to, upon determining that there is no protocol deviation, append a risk indicator to the first reading, the risk indicator based on the determined protocol deviation value.

16. The system of any of claims 13 to 15, wherein the at least one processor (225c) is configured to, upon determining that there is a protocol deviation, cause the one or more first sensor associated with the user device to collect a further first reading.

17. The system of any of claims 13 to 16, further comprising:

generating (225c, 602) a validity range specific to the user based on one or more of the first reading, the second reading, or metadata associated with one or more of the first sensor or the second sensor;
determining (225c) a validity measure based on a comparison of the first reading with the validity range specific to the user; and
determining (604) a measurement validity of the first reading via one or more of the first sensor or the second sensor, wherein determining the measurement validity includes determining whether the validity measure exceeds a validity measure threshold.

18. The system of any of claims 13 to 17, further comprising:

generating (260, 265) a biometric reading based on one or more of the first reading, the second reading, or metadata associated with one or more of the first sensor or the second sensor;
determining a biometric measure based on a comparison of the biometric reading with a patient-specific biometric profile; and
determining a user biometric identification in response to determining that a first reading of a user is requested, wherein determining the user biometric identification includes comparing the biometric measure with a biometric threshold.

19. The system of any of claims 13 to 18, further comprising:

obtaining (225b, 225c) a difficulty score for the first reading based on historical information associated with the first reading, the historical information including one or more of historical information regarding the data indicative of user adherence to the instructions or historical information regarding a number of attempts needed to record the first reading; and
appending a further risk indicator to the first reading, the risk indicator based on the obtained difficulty score.

20. The system of any of claims 13 to 19, further comprising:
determining (225c) an overall risk associated with the first reading, the overall risk determined based on one or more factors with associated weightings.

**FIG. 1A**

EP 4 428 868 A1

FIG. 1B

150

**FIG. 1C**

PATIENT COMMUNICATION DEVICE 205

SECURITY GATEWAY 210

SENSORS 105

102

NETWORK 220

REMOTE PATIENT DATA INGESTION AND STORAGE 215

OTHER SERVERS 225e

PATIENT DATA CONTEXT DEVICE 225a

DATABASE 230

PATIENT DATA RISK EVALUATION DEVICE 225b

PATIENT DATA RISK MANAGEMENT DEVICE 225c

ADMINISTRATOR COMUNICATION DEVICE 225d

OTHER COMMUNICATION DEVICE 225f

**FIG. 2A**

EP 4 428 868 A1

250

PATIENT COMMUNCATION DEVICE 205

SENSOR APPLICATION FRAMEWORK (SAF) 255

INVERSION CONTROL WORKFLOW CONTAINER
295

260

SENSOR LOGIC
SENSOR LOGIC
SENSOR LOGIC
SENSOR LOGIC MANAGEMENT AND DATA INTEGRITY

SENSORS
SENSORS
SENSORS
SENSORS

105

METADATA CONFIGURATION, STORE, AND RETRIEVAL

265

DATA CONFIGURATION, STORE, AND RETRIEVAL

270

DATA AND METADATA SCHEDULING, PACKAGING AND FORWARDING

275

LOCAL, SECURE PATIENT DATA STORE

280

DATA ENCRYPTION AND ACCESS CONTROL

290

EP 4 428 868 A1

**FIG. 2B**

300

DETERMINING THAT A FIRST READING OF A USER CHARACTERISTIC IS REQUESTED

CAUSING A USER DEVICE ASSOCIATED WITH THE USER TO OUTPUT ONE OR MORE INSTRUCTIONS FOR COLLECTING THE FIRST READING

CAUSING ONE OR MORE FIRST SENSOR ASSOCIATED WITH THE USER DEVICE TO COLLECT THE FIRST READING

CAUSING ONE OR MORE SECOND SENSOR ASSOCIATED WITH THE USER DEVICE TO COLLECT A SECOND READING

DETERMINING DATA INDICATIVE OF USER ADHERENCE TO THE INSTRUCTIONS

DETERMINING A PROTOCOL DEVIATION VALUE FOR THE FIRST READING

DETERMINING WHETHER THERE IS A PROTOCOL DEVIATION FOR THE FIRST READING

SAVING THE PROTOCOL DEVIATION DETERMINATION TO A STORAGE DEVICE

# FIG. 3

**FIG. 4**

400

SECURITY GATEWAY — 210

CENTRAL SENSOR SETUP PACKAGE PUBLICATION — 405

NETWORK — 220

PATIENT COMMUNCATION DEVICE 205

SENSOR APPLICATION FRAMEWORK (SAF) 255

SENSOR SETUP
SENSOR SETUP
SENSOR SETUP
SENSOR SETUP
SENSOR SETUP PACKAGE — 410

INSTALLED FOR IOC CONTAINER REFERENCE — 415

INVERSION OF CONTROL WORKFLOW CONTAINER — 295

SENSOR REGISTRATION RECORD — 420

LOCAL, SECURE PATIENT DATA STORE — 280

EP 4 428 868 A1

27

<u>500</u>

| |
|---|
| SELECTING ONE OR MORE SENSOR SETUP PACKAGE (SSP) |

502

| |
|---|
| DEPLOY THE ONE OR MORE SSP TO A SENSOR APPLICATION FRAMEWORK (SAF) |

504

| |
|---|
| OPTIONALLY, GENERATE AN INITIAL REGISTRATION RECORD |

506

# FIG. 5

600

| 602 | DETERMINING NORMALIZATION OF DATA IS REQUESTED |
|---|---|
| 604 | CONDUCTING DATA NORMALIZATION |
| 606 | OPTIONALLY, UPON DETERMINING A NORMALIZATION ISSUE, DETERMINING THAT REPEATING A FIRST READING OF A USER CHARACTERISTIC IS REQUESTED |

# FIG. 6A

# FIG. 6B

700

708

702 — PROCESSOR

724 — INSTRUCTIONS

DISPLAY — 710

704 — MEMORY

724 — INSTRUCTIONS

USER
INPUT/OUTPUT
DEVICE — 712

706 — DRIVE UNIT

722 — COMPUTER
READABLE
MEDIUM

724 — INSTRUCTIONS

COMMUNICATION
INTERFACE — 720

220

NETWORK

**FIG. 7**

Europäisches
Patentamt

European
Patent Office

Office européen
des brevets

# EUROPEAN SEARCH REPORT

**Application Number**

EP 24 16 0976

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/226439 A1 (VERILY LIFE SCIENCES LLC [US]) 27 October 2022 (2022-10-27) * abstract; figure 1 * * paragraph [0001] - paragraph [0004] * * paragraph [0020] - paragraph [0024] * * paragraph [0070] * ----- | 1-20 | INV. G16H10/60 G16H20/30 G16H20/00 |

**TECHNICAL FIELDS SEARCHED (IPC)**

G16H

The present search report has been drawn up for all claims

| Place of search | Date of completion of the search | Examiner |
|---|---|---|
| Munich | 31 May 2024 | Gardiner, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 24 16 0976

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2024

| Patent document cited in search report | Publication date | Patent family member(s) | | Publication date |
|---|---|---|---|---|
| WO 2022226439 A1 | 27-10-2022 | EP | 4327336 A1 | 28-02-2024 |
| | | EP | 4368099 A2 | 15-05-2024 |
| | | JP | 2024517550 A | 23-04-2024 |
| | | WO | 2022226439 A1 | 27-10-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3620937 A1